# EUROPEAN PATENT SPECIFICATION

(11) **EP 4 251 133 B1**
(45) Date of publication and mention of the grant of the patent: **24.09.2025**
(21) Application number: 21807156.1
(22) Date of filing: 23.11.2021
(51) Int. Cl.: A61K 9/06, A61K 9/08, A61K 31/4709, A61K 41/00, A61P 35/00

(54) **COMBINED THERAPY AGAINST CANCER**
KOMBINIERTE KREBSTHERAPIE
THÉRAPIE COMBINÉE CONTRE LE CANCER

(30) Priority: 24.11.2020 EP 20383022
(43) Date of publication of application: 04.10.2023
(73) Proprietor: Universitat Politècnica De Catalunya, 08034 Barcelona (ES)
(72) Inventor: CANAL BARNILS, Cristina, 08034 Barcelona (ES); TORNÍN CAVIELLES, Juan, 33520 Nava (ES)
(74) Representative: Ungria López, Javier
(86) International application number: PCT/EP2021/082591
(87) International publication number: WO 2022/112206

(56) References cited:
- WO-A1-2011/066263
- US-A1- 2019 274 747
- LUO YI ET AL: "Nifuroxazide induces apoptosis, inhibits cell migration and invasion in osteosarcoma", INVESTIGATION NEW DRUGS, MARTINUS NIJHOFF PUBLISHERS, BOSTON, US, vol. 37, no. 5, 25 January 2019 (2019-01-25), pages 1006 - 1013, XP036882679, ISSN: 0167-6997, [retrieved on 20190125], DOI: 10.1007/S10637-019-00724-4

## Description

### Field of the invention

The present invention belongs to the field of Biotechnology and relates to a combined therapy against cancer.

### Background of the invention

Liquids treated with cold atmospheric plasma (CAP) have been used in anti-cancer therapy. Interestingly, CAP has shown to selectively kill cancer cells, without affecting healthy cells. It has been described that the cytotoxicity of the CAP treated liquid depends on the amount of reactive oxygen and nitrogen species (RONS) in it (Bauer, G. et al. Sci Rep 9, 14210 (2019); Tornin, J. et al. Sci Rep 9, 10681, (2019)).

STAT-3 inhibitors have also been assayed in anti-cancer therapy. It has been disclosed the high incidence of STAT3 tyrosine phosphorylation in osteosarcoma cell lines and that targeting STAT3 using STAT3 inhibitor S31-201 in osteosarcoma cell lines showed significant inhibition of cell growth and colony formation, as well as apoptosis enhancement via the caspase-3 pathway *in vitro* (Wang et al. Anticancer Research 34: 6537-6546 (2014)). Luo Yi et al. (2019, Investigation new drugs, Martinus Nijhoff Publishers Boston, US, Vol. 37, no. 5: 1006-1013) discloses the use of the STAT3-inhibitor nifuroxazide for the treatment of osteosarcoma, observing an increased level of ROS.

In anti-cancer therapy, there is still a need to find new treatments that kill cancer cells without affecting healthy cells. CAP has been used and has proven to be effective in different cancer types, including osteosarcoma (OS). OS is the most common pediatric bone primary tumor in the world and the eighth most common childhood cancer. OS is a very aggressive tumor and shows high capacity to metastasize, however current therapies have not advanced much in the last 30 years. Current treatments include a first surgery combined with high doses of methotrexate, cisplatin, doxorubicin or Ifosfamide. Due to the difficult access to surgery resection and the harmful effects of chemotherapy for OS, there is an urgent need to evaluate new treatments that improve both cure and survival in this disease.

### Description of the invention

The present invention provides a solution to the above-mentioned problem. The inventors have surprisingly found that the combination of oxidative stress-based therapies like cold plasma treated liquids or hydrogels with a STAT3 inhibitor is synergistically effective as cancer therapy, dramatically preventing the growth of cancer cells, both cancer stem cells and non-cancer stem cells, while not affecting healthy cells. This allows the use of non-toxic concentrations of both comprising reactive oxygen and nitrogen species (RONS) and a STAT3 inhibitor to achieve a high cytotoxic effect only on cancer cells.

In a first aspect, the present invention relates to a composition or combination comprising:
a. a liquid or a hydrogel comprising RONS; and
b. a STAT3 inhibitor.

In a preferred embodiment, the RONS comprise between 10 and 3000 µM H₂O₂, preferably between 10 and 600 µM H₂O₂, more preferably between 10 and 300 µM H₂O₂, even more preferably between 20 and 250 µM H₂O₂. In another preferred embodiment, the RONS comprise between 10 and 800 µM NO₂⁻, preferably between 10 and 400 µM NO₂⁻, more preferably between 10 and 250 µM NO₂⁻, even more preferably between 20 and 250 µM NO₂⁻. In a preferred embodiment, the RONS comprise between 10 and 3000 µM H₂O₂ and/or between 10 and 800 µM NO₂⁻. In another preferred embodiment, the RONS comprise between 10 and 600 µM H₂O₂ and/or between 10 and 400 µM NO₂⁻. In another preferred embodiment, the RONS comprise between 10 and 300 µM H₂O₂ and/or between 10 and 250 µM NO₂⁻. In another preferred embodiment, the RONS comprise between 20 and 250 µM H₂O₂ and/or between 20 and 250 µM NO₂⁻.

The RONS concentration is quantified either using the AR/HRP reagent method or the Griess reagent method for H₂O₂ and NO₂⁻, respectively. Also, plastic strips with test paper which allow quantification of H₂O₂ based on a redox reaction and NO₂⁻, also using the Griess reagent may be used when a hydrogel is used and the protein solution causes interferences with the AR/HRP reagent method or the Griess reagent method. These two methods give equivalent results.

In a preferred embodiment, the composition or combination comprises a liquid comprising RONS, wherein the liquid is an aqueous medium. The aqueous medium may be selected from water, saline aqueous solutions such as Ringer's solution, parenteral solution for hospital use, solutions used as drug vehicles or cell culture media.

In a preferred embodiment, the composition or combination comprises an hydrogel comprising RONS, wherein the hydrogel is an aqueous solution comprising at least one of gelatin, a gelatin derivative such as metacrylated gelatin, fibrin, fibronectin, collagen, a collagen derivative, alginate, agarose, cellulose, modified cellulose such as hydroxypropyl cellulose, carboxymethylcellulose or hydroxyethyl cellulose, xantan gum, polyethyleneglycol, hyaluronic acid, chitosan, polylactide-co-glycolide, polyhydroxyalcanoates. In a preferred embodiment, the hydrogel is an aqueous solution comprising gelatin, alginate, collagen or mixtures thereof.

In a preferred embodiment, the composition or combination further comprises a ceramic material comprising calcium. Preferably, the ceramic material comprising calcium is selected from calcium phosphate, hydroxyapatite, calcium deficient hydroxyapatite, brushite, fluorapatite, calcium- sodium and potassium- phosphate, calcium- and sodium-phosphate, calcium- and potassium- phosphate, calcium pyrophosphate, calcium carbonate, calcium sulphate, calcium sulphate hemihydrate, calcium oxide, calcium hydroxide, and mixtures thereof, preferably the ceramic material is hydroxyapatite, brushite, tricalcium phosphate or mixtures thereof.

In a preferred embodiment, the STAT3 inhibitor prevents Stat3 expression, STAT3 phosphorylation, STAT3 dimerization, STAT3 translocation to the nucleus, STAT3 DNA binding or STAT3 mediated transcription. In a preferred embodiment, the STAT3 inhibitor prevents STAT3 phosphorylation, more preferably the STAT3 inhibitor prevents STAT3 phosphorylation at tyrosine 705. The STAT3 inhibitor can be selected from S3I-201, WP1066, Resveratrol, Stattic, Niclosamide, STAT3-IN-1, STAT5-IN-1, AS1517499, C188-9, BP-1-102, SH-4-54, Cryptotanshinone, Bosutinib (SKI-606), Fludarabine, Nifuroxazide, Brevilin A, RCM-1, Kaempferol-3-O-rutinoside, Cucurbitacin IIb, SC-43, Scutellarin, HJC0152, SH5-07 (SH-5-07), APTSTAT3-9R, Ochromycinone (STA-21), Napabucasin (BBI608), HO-3867, Artesunate or any combination thereof. In a preferred embodiment, the STAT3 inhibitor is S31-201 or BBI608. In a preferred embodiment, the STAT3 inhibitor is S31-201. In another preferred embodiment, the STAT3 inhibitor is BBI608.

In a preferred embodiment, the composition or combination of the invention further comprises at least an active agent selected from a chemotherapeutic agent and an immunotherapeutic agent.

A second aspect of the present invention relates to the composition or combination of the first aspect for use in the treatment of cancer. In a preferred embodiment, the cancer is selected from bone cancer, sarcoma, prostate cancer, urotelioma, breast cancer, brain cancer, or colon cancer. In a more preferred embodiment, the bone cancer is osteosarcoma.

In a preferred embodiment of the second aspect, the liquid or hydrogel comprising RONS and the STAT3 inhibitor are administered simultaneously or sequentially. In another preferred embodiment, the composition is administered before or after surgery.

In another aspect, the present invention relates to a method of treating cancerous tissue in a subject comprising administering to the subject a composition or combination comprising:
(a) a liquid or a hydrogel and reactive oxygen and nitrogen species (RONS); and
(b) a STAT3 inhibitor.

In a preferred embodiment, components (a) and (b) are administered either simultaneously or subsequently.

In another aspect, the present invention relates to a method of treating cancerous tissue in a subject comprising applying cold atmospheric plasma to said tissue and administering a STAT3 inhibitor to said subject. The cancerous tissue is preferably osteosarcoma.

### Brief description of the drawings

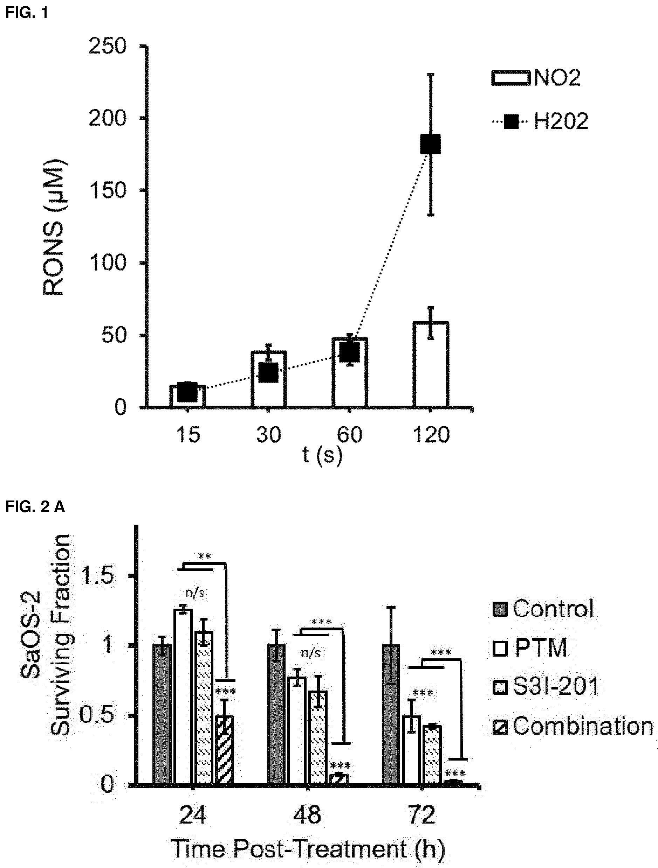

**Figure 1****.** Concentration of RONS (µM) in plasma treated liquid, where squares indicate micromolar (µM) concentration of Hydrogen Peroxide (H₂O₂) by AmplexRed/HRP assay and columns indicate the micromolar (µM) concentration of Nitrites (NO₂⁻) by Griess assay. Data are presented as mean, n=3.

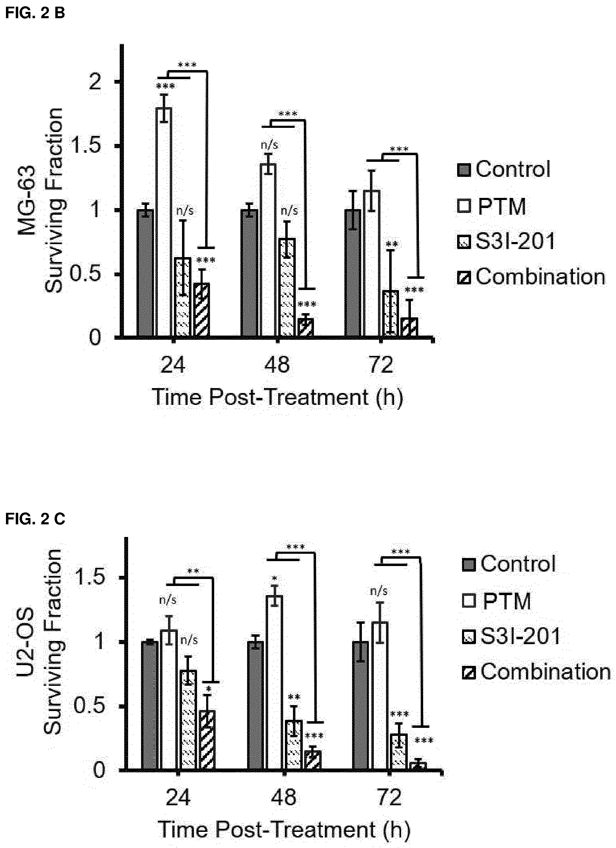

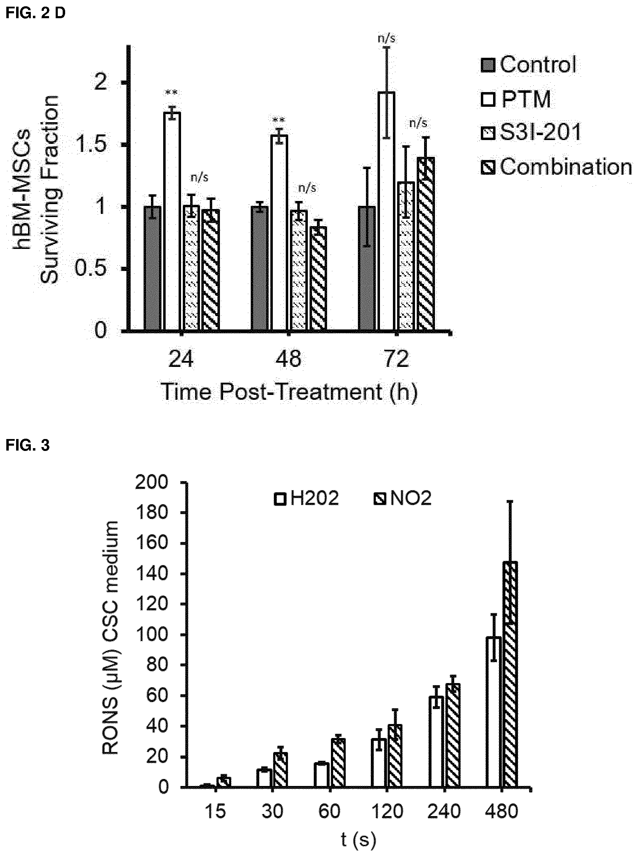

**Figure 2****.** Synergic effect of the combined therapy on cell viability. Cell viability (WST1 assay) measured after the treatment of the indicated cell lines (A. SaOS-2. B. MG-63. C. U2-OS. D. hBM-MSCs) using 15s - plasma treated medium (PTM), S31-201 (80 µM) or a combination of both for 24, 48 and 72 hours. For each time, the four columns from left to right correspond to control, plasma treated medium (PTM), S3I-201 and combination of plasma treated medium and S3I-201. Cell viability is expressed relative to the corresponding control. Data represent the mean and standard deviation of n=3 independent experiments.
**Figure 3****.** Concentration of RONS in CSC culture. Columns indicate micromolar (µM) concentration of Hydrogen Peroxide (H₂O₂) by AmplexRed/HRP assay and markers the micromolar (µM) concentration of Nitrites (NO₂⁻) by Griess assay on indicated treatment times. Data are presented as mean, n=3.

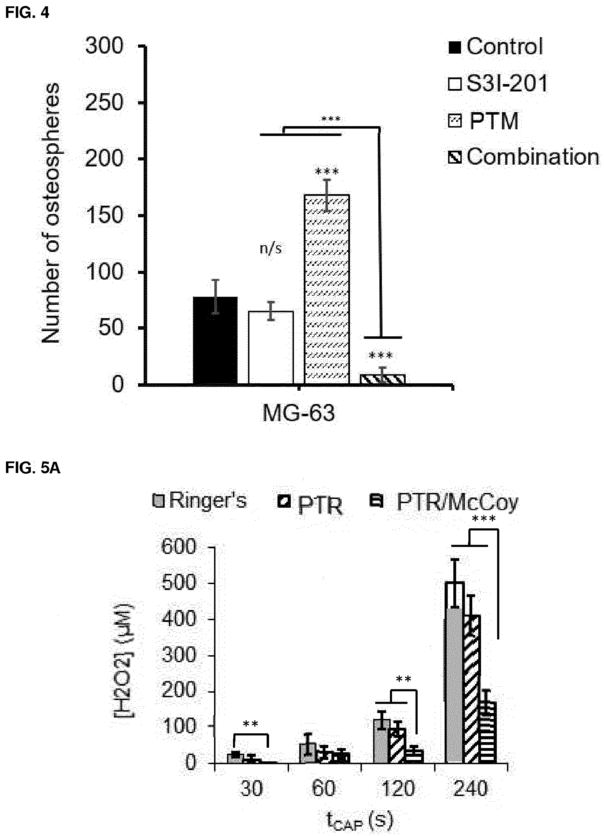

**Figure 4****.** STAT3 inhibitor and plasma treated medium act synergistically preventing the growth of osteosarcoma cell line derived tumorspheres. Tumorsphere number from MG-63 cells. n=3 independent replicates, and each replicate from n=6 independent cultures.

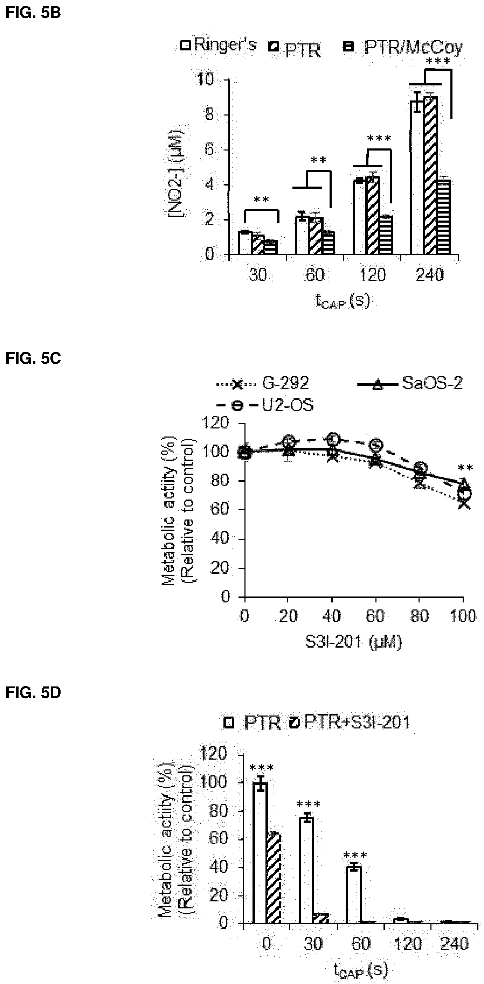

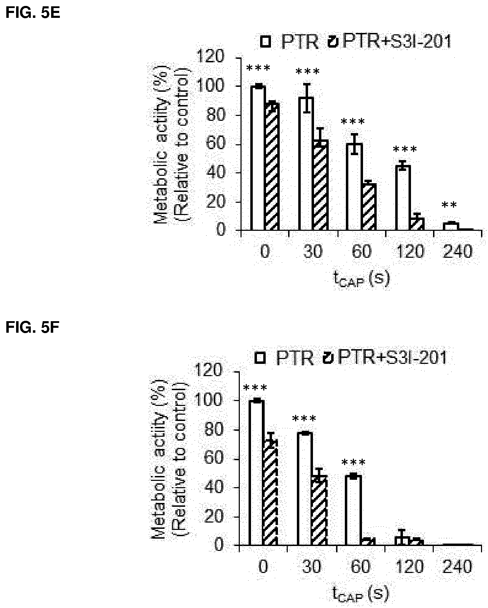

**Figure 5****.** Synergistic effect of PAR with S31-201 reducing cell viability in OS cell lines in adherent culture. Concentration of H₂O₂ (A) and NO₂⁻ (B) measured in plasma-treated Ringer's saline (PTR) before and after the addition of 10% of FBS and after diluting 1:1 in McCoy's A5 Medium Modified. G-292, SaOS-2 and U2-OS cells in adherent culture were exposed during 2 hours (C) to different concentrations of S31-201 (20-100 µM) in untreated PTR and to (D-F) PTR treated for 30-240 seconds with and without the addition of 100 µM of S31-201 after treatment. After that, PTR was diluted 1:1 in McCoy's A5 Medium Modified. Metabolic activity was determined 72 hours after PTR exposure by PrestoBlue assay. Values were relativized to cells exposed to untreated PTR.

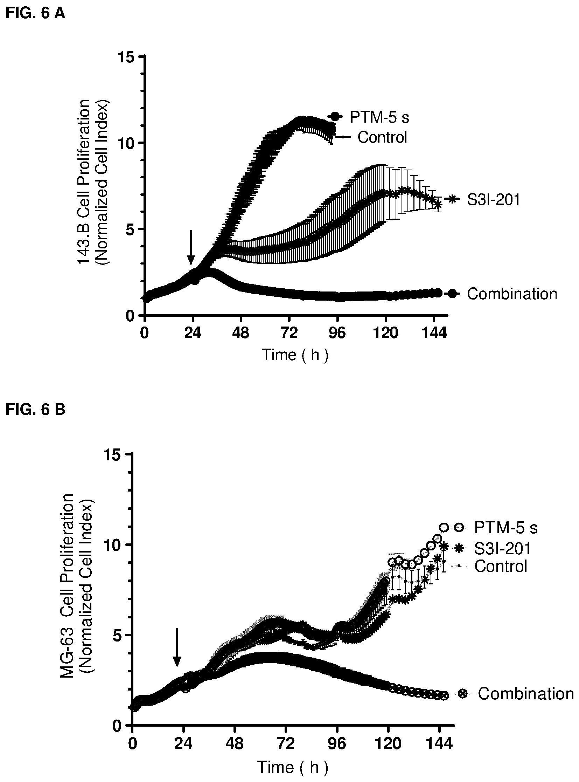

**Figure 6****.** Synergic effect of the combined therapy on cell proliferation ability in Osteosarcoma cells 143.B (A) and MG-63 cells (B). Real-time cell proliferation of osteosarcoma cells 143.B (A) and MG-63 (B) with a treatment of 5 s with PTM (plasma treated cell culture medium, PTM-5 s), 100 µM of S3I-201, or the combination of both. Data shows the Normalized Cell Index relative to initial time (t = 0 h) up to final step (t = 144 h). Black arrow indicates the treatment addition (t = 24 h) post-seeding.

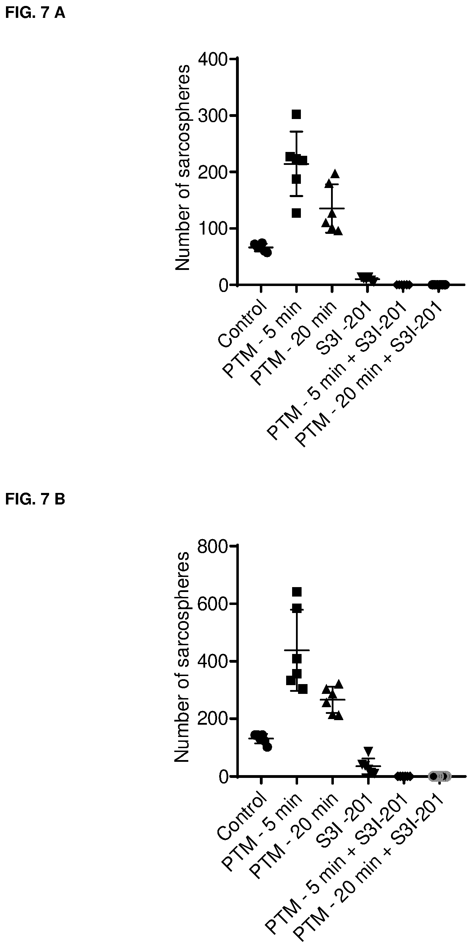

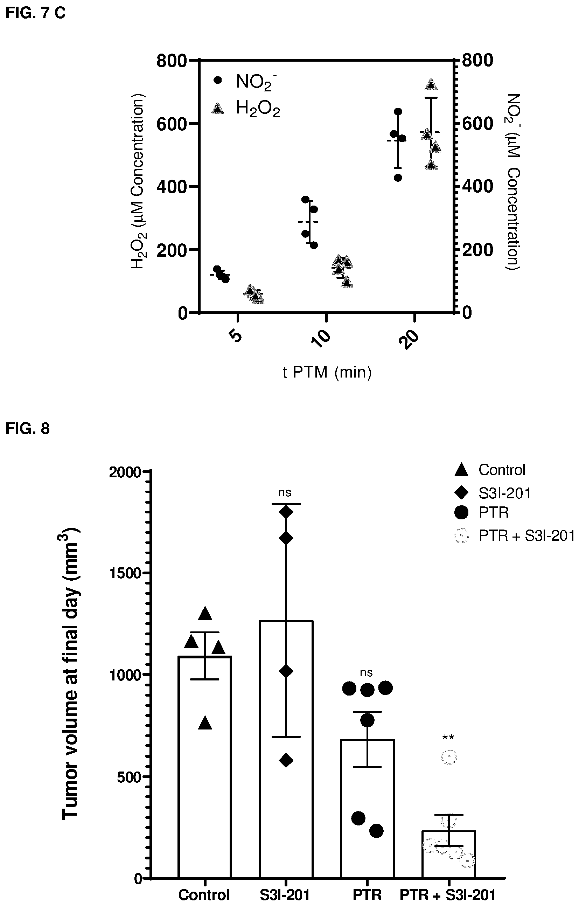

**Figure 7****.** Synergic effect of the combined therapy on sarcosphere number reduction. The (A) MG-63 sarcospheres and (B) 143.B sarcospheres formed were scored (size ≥70 µM) and counted. Data represent the number of sarcospheres formed as mean and standard deviation of n= 6 independent experiments (*p < 0.01; **p < 0.001; ****p < 0.0001 one-way ANOVA). (C) Concentration of NO₂⁻ and H₂O₂ in micromolar concentration in PTM 5, 10 or 20 min.
**Figure 8****.** Synergistic effect of plasma treated medium and S3I-201 in reducing osteosarcoma tumor size *in vivo.* Tumor volume of the different groups at final day (when tumors reach 1000 mm³). The one-way ANOVA was performed to determine the statistical significance between control and treated groups. We found that mice treated with PTM alone (n=5) or S31-201 (n=4) display a tumor volume in a similar way that control (n=4) group, however the volume of tumors in combination group PTM+ S3I-201 (n=6) was significantly lower than control (**p < 0.0065).

### Examples

The following examples are provided to further illustrate, but not to limit this invention.

### 1. Plasma treatment and RONS concentration

The generation of RONS in the cell culture medium following plasma treatment is time dependent and shows an equilibrated quantity of NO₂⁻ and H₂O₂ (Figure 1). Most treatment times do not lead to significant differences on the concentration of NO₂⁻ except for 120s where the concentration of H₂O₂ generated in plasma treated medium is up to 3 times higher than NO₂⁻ (Figure 1). These concentrations are the ones used in the next example, where 15s-plasma treated medium was used.

### 2. S3I-201 and plasma treated medium act synergistically preventing the growth of osteosarcoma cell lines but do not affect healthy cells

The viability of osteosarcoma SaOS-2, MG-63 and U2-OS cells and healthy hBM-MSCs is shown in Figure 2. 15s-plasma treated medium was cytotoxic only to SaOS-2 cells whereas MG-63, U2-OS and hBM-MSCs cells showed an increase in cell proliferation (Figures 2 A-D). Surprisingly, the combination of 15s-plasma treated medium and the STAT3 inhibitor S31-201 significantly showed a synergistically cytotoxic effect than separate treatments. The combined treatment increased the cytotoxicity of plasma treated medium in the three osteosarcoma cell lines even at very low dose of plasma treated medium-15s, while healthy cells were not affected (Figures 2 A-D).

### 3. S3I-201 and plasma treated medium prevents growth of cancer stem cell (CSC) cultures.

The cytotoxic potential of a plasma treated medium (DMEM-F12) was tested over 3D monoclonal osteospheres. The generation of RONS in the cell culture medium following plasma treatment is time dependent and shows an equilibrated cocktail of NO₂⁻ and H₂O₂ in all treatment times investigated (Figure 3).

We directly treated already formed osteospheres of MG-63 cells at day 7 post-seeding with a combination of 480s-plasma treated medium with STAT3 inhibitor S31-201 for 3 days. Notably, the combination of S3I-201 and 480s-plasma treated medium, was completely effective reducing the number of osteospheres (Figure 4).

### 4. Synergistic effect of plasma treated medium with S3I-201 reducing cell viability in OS cell lines in adherent culture.

Concentration of H₂O₂ (Figure 5A) and NO₂⁻ (Figure 5B) were measured in plasma-treated Ringer's saline (PTR) before and after the addition of 10% of FBS and after diluting 1:1 in McCoy's A5 Medium Modified. G-292, SaOS-2 and U2-OS cells in adherent culture were exposed during 2 hours to (Figure 5C) different concentrations of S31-201 (20-100 µM) in untreated PTR and to (Figures 5D-F) PTR treated for 30-240 seconds with and without the addition of 100 µM of S3I-201 after treatment. After that, PTR was diluted 1:1 in McCoy's A5 Medium Modified. Metabolic activity was determined 72 hours after PTR exposure by PrestoBlue assay. Values were relativized to cells exposed to untreated PTR.

### 5. Cytotoxic effect of plasma treated hydrogel solutions

A 50/50 blend of 0.5 weight % alginate and 2 weight % gelatin solutions were prepared (final concentration of 0.25 % wt alginate and 1 % wt gelatin). The mixture of alginate/gelatin was prepared is by vortexing in a ratio 1:1, 2 % wt gelatin with 0.5 % wt alginate for 2 minutes. Gelatin in powder is mixed with MilliQ water at 37 °C using magnetic stirring for 2 hours to obtain a 2 % wt gelatin gel. 0.5% alginate was prepared by mixing alginate powder with MilliQ water using a SpeedMixer^{™} DAC 150.1 FVZ-K (SpeedMixer^{™}, Germany) at 3500 r.p.m. for 15 min. The 0.25 % wt alginate and 1 % wt gelatin aqueous mixture was treated with an atmospheric pressure plasma jet kINPen IND^{®} (Neoplas, Germany) operating with Argon to generate plasma. Treatment conditions: 1 L/min gas flow, 10 mm nozzle distance, and 180 seconds treatment. Treatment performed in 200 µL of mixture in a 96-well plate. Said plasma-treated mixture produced the following concentrations of reactive species in the material:

| | **H₂O₂ (mg/L)** | **NO₂⁻ (mg/L)** | **NO₃⁻ (mg/L)** |
|---|---|---|---|
| **Water** | **10.3** | **2.6** | **-** |
| **Hydrogel solution** | 16.7 | 17.0 | 124.0 |

As shown in the table, the values of reactive species obtained in this composition are several-fold higher than those generated in water. Said plasma-treated mixture was used in cell viability assays in both an osteosarcoma cell line (SaOS-2) and in healthy cells (human bone marrow mesenchymal stem cells or hBM-MSC):

| **Hydrogel solution** | **Cell viability at 72 h (%)** |
|---|---|
| **SaOS-2** | 40.94 ± 3.44 |
| **hBM-MSC** | 90.57 ± 8.19 |

This composition shows selectivity of the plasma-treated polymer solution on the cancer cell line, allowing the survival of healthy cells (hBM-MSC) after 72 hours.

### 6. Cytotoxic effect of plasma treated hydrogel solutions with ceramic material

The composition of the preceding example was prepared by treating with plasma during 5 minutes instead of 3 minutes, and further comprising 5 % wt of calcium deficient hydroxyapatite microspheres (MS), which were added and mixed in the vortex for 2 min. The diameter of the microspheres was 100 µm<Ø<150 µm. The amount of RONS was not affected by the addition of the bioceramic material. The concentration of reactive species generated by plasma in the polymer solution and in the composition after adding the bioceramic material is equivalent, as can be seen below:

| **Example** | **[H₂O₂] (mg/L)** | **[NO₂⁻] (mg/L)** | **[NO₃⁻] (mg/L)** |
|---|---|---|---|
| **Hydrogel solution** | 78.0 ± 15.6 | 20.0 ± 4.0 | 297.0 ± 59.4 |
| **Hydrogel solution** + **5% microspheres** | 84.7 ± 16.9 | 21.5 ± 4.3 | 270.0 ± 54.0 |

The species generated in the composition can be released to a surrounding media and preserved at least for 24 hours. This was also tested with a composition where the MS were previously loaded with the active agent doxorubicin (DOX):

| | **H₂O₂ concentration in 1 mL release media (mg/L)** | | |
|---|---|---|---|
| **Time (h)** | **Hydrogel** | **Hydrogel+MS** | **Hydrogel with DOX-loaded MS** |
| **0** | 0 | 0 | 0 |
| **0.5** | 2.37 ± 0.15 | 3.11 ± 0.19 | 3.18 ± 0.11 |
| **1** | 2.55 ± 0.37 | 4.18 ± 0.39 | 2.57 ± 0.09 |
| **2** | 1.99 ± 0.34 | 3.50 ± 0.25 | 2.64 ± 0.09 |
| **4** | 2.08 ± 0.33 | 3.76 ± 0.39 | 3.06 ± 0.10 |
| **24** | 1.95 ± 0.23 | 3.06 ± 0.64 | 2.21 ± 0.08 |

| | **NO₂⁻ concentration in 1 mL release media (mg/L)** | | |
|---|---|---|---|
| **Time (h)** | **Hydrogel** | **Hydrogel+MS** | **Hydrogel with DOX-loaded MS** |
| **0** | 0 | 0 | 0 |
| **0.5** | 0.25 ± 0.02 | 0.25 ± 0.03 | 0.25 ± 0.05 |
| **1** | 0.31 ± 0.02 | 0.36 ± 0.04 | 0.29 ± 0.06 |
| **2** | 0.38 ± 0.01 | 0.46 ± 0.01 | 0.35 ± 0.07 |
| **4** | 0.43 ± 0.02 | 0.51 ± 0.03 | 0.29 ± 0.06 |
| **24** | 0.54 ± 0.06 | 0.60 ± 0.04 | 0.32 ± 0.06 |

Said Hydrogel + MS was used in cell viability assays in osteosarcoma cell line (SaOS-2):

| | **SaOS-2 cell viability at 24 h (%)** | **SaOS-2 cell viability at 72 h (%)** |
|---|---|---|
| **Untreated composition** | 93.6 ± 6.8 | 96.7 ± 2.1 |
| **Hydrogel** + **MS** | 13.8 ± 1.3 | 7.5 ± 5.5 |

### 7. Synergistic effect of plasma treated medium with S3I-201 reducing cell viability in OS cell lines in adherent culture.

Cell proliferation was studied using the xCELLigence system (ACEA Biosciences, Inc, San Diego, CA, USA). Osteosarcoma cells 143.B (Figure 6A) and MG-63 (Figure 6B) were seeded in specially designed microtiter plates containing interdigitated gold microelectrodes at a density of 1 × 10⁴ in 500 µL of culture medium. On the following day, 400 µL of culture medium was replaced with 400 µL of PTM-5 s, S31-201 100 µM, and the combination of both. Real-time proliferation measured as cell impedance changes was monitored by the xCELLigence system every hour until the end of experiment. Figures 6 A and B show the Normalized Cell Index relative to initial time (t = 0 h) up to final step (t = 144 h). The black arrow indicates the treatment addition (t = 24 h) post-seeding. The results clearly show that the combination therapy of PTM and STAT3 inhibitor are acting synergistically to abrogate cell proliferation.

### 8. Synergistic effect of plasma treated medium with S3I-201 eliminating osteosarcoma cancer stem cells

Osteosarcoma cells MG-63 and 143.B were cultured in sarcosphere-forming conditions (Cancer Stem Cell (CSC) isolation standard method) for 3 days and then were incubated for another 7 days in contact with PTM according to the following parameters: Helium flow rate: 1 L/min; Gap between APPJ nozzle and CSC medium surface: 10 mm; Treatment times: between 5 and 20 minutes. The CSC Medium was DMEM/F-12 supplemented with GlutaMAX^{™} (1X; GibcoTM, cat.no 10565018, Carlsbad, CA, USA), B-27 Supplement (1:50; Life Technologies), Heparin (1:1000; Sigma), human bEGF (20 ng/ml), human bFGF (10 ng/ml; GoldBio) and sodium pyruvate. The sarcospheres were treated at day 3 with PTM (5 and 20 min), with S31-201 (100 µM) or with the combination of both for 7 days more.

The viability of sarcospheres at day 10 consistently showed that self-renewal ability and tumorsphere growth increased upon treatment with PTM for 5 and 20 min in MG-63 and 143.B cells (Figs. 7 A and B). The number of sarcospheres was significantly reduced by S31-201 inhibitor alone (*p < 0.01) and sarcospheres were completely eradicated using the combination of PTM + S31-201 (*p < 0.001) (Figs. 7A and B). Figures 7 A and B show the ability of the PTM in combination with S31-201 to eliminate the Cancer Stem Cells (CSC) in Osteosarcoma.

Figure 7C shows the RONS concentrations in the PTM used for the treatment of the sarcospheres. Non-significant differences were recorded among nitrites (Griess Reagent Assay) or peroxides (Amplex Red Assay) in PTM. The concentration of NO₂⁻ ranging from 119 ± 13 µM to 545 ± 57 µM in PTM - 5 min and 20 min respectively. The same trend was observed with peroxides, ranging from 60 ± 11 µM to 572 ± 109 µM in PTM-5 min and 20 min respectively.

These data clearly confirmed that only the combinatory treatment powerfully eliminates CSC survival. CSC are a subpopulation of cells directly related to tumor relapse metastases and chemoresistance and there is an urgent need for therapies that eliminate CSC. These results show that the combined therapy with PTM and the STAT3 inhibitor acts synergically to eliminate these CSC.

### 9. Synergistic effect of plasma treated medium and S3I-201 in reducing osteosarcoma tumor size in vivo

To investigate the role PTM in combination with S3I-201 in osteosarcoma disease development, we used a murine orthotopic osteosarcoma model in which human 143B osteosarcoma cells are injected into the tibia of immunodeficient mice. All *in vivo* tumor experiments were performed with female nude mice (NMRI-Foxn1nu/nu mice from Janvier Labs). For orthotopic primary tumor growth, 50,000 cells were injected into the right tibia of 7 weeks old mice under isoflurane anaesthesia. Mice received a daily para-tumoral injection of 100 µL of PTM - 20 min or 5 mg/kg S31-201 (orally, dissolved in corn oil) three times per week, or a combination of both treatments. In parallel, control group received a daily para-tumoral injection of Ringer's Saline or 200 µL corn oil orally. Primary tumors were detected and quantified by bioluminescent *in vivo* imaging five days day after cell injection and subsequently once a week until the tumors reached the study endpoint (termination criterion: primary tumor total flux > 108 photons/s). Figure 8 shows the mean tumor volume differences between groups at final day (when tumors reach 1,000 mm³), which were determined using a caliper or measuring the luminescence intensity using an IVIS Spectrum (Caliper Life Sciences, Hopkinton, MA). The one-way ANOVA was performed to determine the statistical significance between control and treated groups. We found that mice treated with PTR alone (n=5) or S3I-201 (n=4) display a tumor volume in a similar way that control (n=4) group. However, the volume of tumors in the combination group PTM+ S31-201 (n=6) was significantly lower than control (**p < 0.0065). Taken together, these results indicate that only the combination of PTM and S31-201 induces an anti-tumoral effect *in vivo* against osteosarcoma.

### Materials and Methods

### Cell culture and drugs

We evaluated the effects of cold plasma treated medium on the osteosarcoma cell lines SaOS-2, MG-63 and U2-OS vs healthy hBM-MSCs (both cell types obtained from ATCC, USA). In this study cell lines were grown in Dulbecco's Modified Eagle Medium (DMEM) with glucose (4,5g/L), pyruvate, no glutamine (Gibco^{™}, USA), with 10% fetal bovine serum (FBS) (Gibco^{™} cat no. 10270098, USA), 2mM L-glutamine (Gibco^{™}), 100 units/mL penicillin (Gibco^{™}) and 100 µg/mL streptomycin (Gibco^{™}). The cells were incubated at 37 °C, 95% humidity and 5% CO₂. Also, G-292, SaOS-2 and U2-OS cells were cultured in McCoy's A5 Medium Modified with 1,5 mM L-glutamine (GibcoTM, Carlsbad, CA, USA) supplemented with 10% of fetal bovine serum (FBS), penicillin/streptomycin (50 U/mL and 50 µg/mL, respectively) and 1 mM sodium pyruvate, all from GibcoTM. S31-201 (cat. No S1155) was obtained from Selleckhem.

### Cold-plasma jet device and application to culture medium or monolayer culture.

kINPen^{®} IND (Neoplas tools GmbH, Greifswald, Germany) is a commercial plasma jet used in clinics that consists of a hand-held unit that discharges plasma under atmospheric conditions, employing a DC power unit and Argon gas to generate the plasma. In the centre of a ceramic capillary (inner diameter 1.6 mm) a pin-type electrode (1 mm diameter) is mounted, and a ring around the dielectric as grounded counter-electrode. The needle is powered by a small RF generator producing a sinusoidal voltage waveform ranging from 2 kV to 3 kV amplitude peak at a frequency of 1 MHz and modulated with 2.5 kHz and a plasma duty cycle of 1:1.

To apply the plasma directly or to treat the medium with the plasma, we designed a protocol that allowed us compare between methods under the same operation parameters: Argon flow of 3 L/min, at a distance of 10 mm between the surface of the liquid and the jet nozzle, during 15,30,60, 120, 240 or 480 seconds over 1mL of culture medium (for example, DMEM, high glucose, no glutamine, no phenol red without 0.1g/L Sodium Pyruvate (GibcoTM , cat.no 11360070, Carlsbad, CA, USA), or DMEM-F12) in a 24-well plate containing 30.000 cells/well.

To produce the plasma-treated Ringer's saline (PTR), 2 mL of sterile Ringer's saline (8.6 g/L NaCl, 0.33 g/L CaCl₂ and 0.3 g/L KCI) were placed under the plasma jet at room temperature with a gas flow of 3 L/min and a distance of 10 mm from the jet nozzle in sterile conditions. The liquid was placed in multiple well plates of 1.9 cm² of surface (24 well-plates). Plasma treatment times between 30-240 seconds were investigated. A 10% of FBS was added immediately after treatment.

### Metabolic Activity

Subconfluent G-292, SaOS-2 and U2-OS cells were trypsinized, centrifuged and seeded in 48-well plates at a density of 15x 10³ cells/well, and incubated in 300 µL of their corresponding medium for 24 h. On the one hand, the culture medium was replaced in each cell line after incubation by 300 µL of Ringer's with 10% of FBS with different concentrations (20-100 µM) of S31-201. On the other hand, the culture medium was replaced in each cell line by 300 µL of PTR treated during 30-240 seconds with and without S31-201 (100 µM). Cells were incubated during two hours for each condition in triplicate. As a positive control, each cell line was incubated during two hours with Ringer's supplemented with 10% of FBS. Afterwards, 300 µL of corresponding fresh medium was added in each condition. Cells were then incubated at 37 °C for 72 hours. Cell metabolism was evaluated by PrestoBlue assay; 20% of PrestoBlue reagent in culture media were employed. As a negative control, PrestoBlue was incubated without cells. Fluorescence were measured with λex/em of 530/590 nm and fluorescence from negative control was subtracted. Fluorescence of each treated condition was referenced to positive control.

### Determination of RONS

We determined the concentration of Hydrogen Peroxide, and Nitrites in plasma treated culture media, following the same protocol described before in Tornin, J. et al. Sci Rep 9, 10681, (2019). Briefly, nitrite concentration was performed using Griess reagent and the concentration of hydrogen peroxide was determined by a redox reaction using a coloured reagent and Horseradish Peroxidase.

### Monolayer cell viability assay and Immunofluorescence

To evaluate the antitumor effects, a WST-1 (Roche, Germany) cell proliferation assay was performed according to the manufacturer's instructions. Cells were seeded in a 24-well plate at a density of 30×10³ cells per 1000µL of culture medium. On the following day, the culture medium was replaced with 1000µL of plasma treated medium. After 24.48 or 72 hours, WST-1 working solution (18µL/mL) was added to each well and plates were incubated at 37°C for 60 min. Absorbance was measured at λ_{abs} =440 nm. Each experiment was performed by independent triplicates. Medium untreated with cold plasma was used as control.

### Culture of osteospheres and viability

MG-63 cells line was plated at a density of 1.500 cells per well in UltraLow Costar 6-well plates (Corning) to prevent cell attachment, in serum-free sphere medium containing DMEM-F12+Glutamax (Gibco), B-27/VitA Supplement (1:50; Life Technologies), Heparin (1:1000; Sigma), the growth factors human EGF (20 ng/ml) and human bFGF (10 ng/ml; GoldBio) and 1 % methylcellulose (Sigma) to avoid cell aggregation. In addition, fresh aliquots of EGF and bFGF were added every three days. To analyze the effects of plasma treated media or STAT3 inhibitor *in vitro,* we treated sphere cultures at day 7 and then we grew them in tumorspheres culture conditions to assay the ability of the drug to inhibit the formation of tumorspheres for 72 h.

### Statistics

For the statistics analysis in the results shown in the figures, 95 % confidence intervals were determined, calculating the mean and standard deviation of the 3 independent experiments. Student T test was used for determining significant differences comparing each treatment with control (untreated). Also, the combination was found to be significant compared to plasma treated medium and to S31-201 treatment alone. One-way ANOVA analysis is indicated in the figure where * means *p < 0.05;* ** means *p < 0.01* and *** means *p* < *0.001).*

## Claims

1. A composition or combination comprising:
a. a liquid or a hydrogel comprising reactive oxygen and nitrogen species (RONS);
and
b. a STAT3 inhibitor.

2. The composition or combination according to the preceding claim, wherein the RONS comprise between 10 and 3000 µM H₂O₂, preferably between 10 and 600 µM H₂O₂, more preferably between 10 and 300 µM H₂O₂, even more preferably between 20 and 250 µM H₂O₂.

3. The composition or combination according to any one of the preceding claims, wherein the RONS comprise between 10 and 800 µM NO₂⁻, preferably between 10 and 400 µM NO₂⁻, more preferably between 10 and 250 µM NO₂⁻, even more preferably between 20 and 250 µM NO₂⁻.

4. The composition or combination according to any one of the preceding claims, wherein the liquid is an aqueous medium.

5. The composition or combination according to any one of the preceding claims, wherein the hydrogel is an aqueous solution comprising at least one of gelatin, a gelatin derivative such as metacrylated gelatin, fibrin, fibronectin, collagen, a collagen derivative, alginate, agarose, cellulose, modified cellulose such as hydroxypropyl cellulose, carboxymethylcellulose or hydroxyethyl cellulose, xantan gum, polyethyleneglycol, hyaluronic acid, chitosan, polylactide-co-glycolide, polyhydroxyalcanoates.

6. The composition or combination according to any one of the preceding claims, wherein the hydrogel is an aqueous solution comprising gelatin, alginate, collagen or mixtures thereof.

7. The composition or combination according to any one of the preceding claims, further comprising a ceramic material comprising calcium.

8. The composition or combination according to the preceding claim, wherein the ceramic material comprising calcium is selected from calcium phosphate, hydroxyapatite, calcium deficient hydroxyapatite, brushite, fluorapatite, calcium-sodium and potassium- phosphate, calcium- and sodium- phosphate, calcium- and potassium- phosphate, calcium pyrophosphate, calcium carbonate, calcium sulphate, calcium sulphate hemihydrate, calcium oxide, calcium hydroxide, and mixtures thereof, preferably the ceramic material is hydroxyapatite, brushite, tricalcium phosphate or mixtures thereof.

9. The composition or combination according to any one of the preceding claims, wherein the STAT3 inhibitor prevents STAT3 expression, STAT3 phosphorylation, STAT3 dimerization, STAT3 translocation to the nucleus, STAT3 DNA binding or STAT3 mediated transcription.

10. The composition or combination according to any one of the preceding claims, wherein the STAT3 inhibitor prevents STAT3 phosphorylation, more preferably the STAT3 inhibitor prevents STAT3 phosphorylation at tyrosine 705.

11. The composition or combination according to any one of the preceding claims, wherein the STAT3 inhibitor is S3I-201 or BBI608.

12. The composition or combination according to any one of the preceding claims, further comprising at least an active agent selected from a chemotherapeutic agent and an immunotherapeutic agent.

13. The composition or combination according to any one of the preceding claims for use in the treatment of cancer.

14. The composition or combination for use according to the preceding claim, wherein the cancer is selected from bone cancer, prostate cancer, breast cancer, brain cancer, or colon cancer.

15. The composition or combination for use according to the preceding claim, wherein the bone cancer is osteosarcoma.

16. The composition or combination for use according to any one of claims 13 to 15, wherein the administration of the liquid or a hydrogel comprising reactive oxygen and nitrogen species is simultaneous or sequential with respect to the administration of the STAT3 inhibitor.

## Patentansprüche

1. Zusammensetzung oder Kombination, umfassend:
a. eine Flüssigkeit oder ein Hydrogel, umfassend reaktive Sauerstoff- und Stickstoffspezies (RONS); und
b. einen STAT3-Inhibitor.

2. Zusammensetzung oder Kombination nach dem vorhergehenden Anspruch, wobei die RONS zwischen 10 und 3000 µM H₂O₂, bevorzugt zwischen 10 und 600 µM H₂O₂, bevorzugter zwischen 10 und 300 µM H₂O₂, noch bevorzugter zwischen 20 und 250 µM H₂O₂ umfassen.

3. Zusammensetzung oder Kombination nach einem der vorhergehenden Ansprüche, wobei die RONS zwischen 10 und 800 µM NO₂⁻, bevorzugt zwischen 10 und 400 µM NO₂⁻, bevorzugter zwischen 10 und 250 µM NO₂⁻, noch bevorzugter zwischen 20 und 250 µM NO₂⁻ umfassen.

4. Zusammensetzung oder Kombination nach einem der vorhergehenden Ansprüche, wobei die Flüssigkeit ein wässriges Medium ist.

5. Zusammensetzung oder Kombination nach einem der vorhergehenden Ansprüche, wobei das Hydrogel eine wässrige Lösung ist, umfassend mindestens eines von Gelatine, einem Gelatinederivat wie etwa methacrylierter Gelatine, Fibrin, Fibronektin, Kollagen, einem Kollagenderivat, Alginat, Agarose, Cellulose, modifizierter Cellulose wie etwa Hydroxypropylcellulose, Carboxymethylcellulose oder Hydroxyethylcelloluse, Xanthan, Polyethylenglycol, Hyaluronsäure, Chitosan, Polylactid-co-Glycolid, Polyhydroxyalkanoaten.

6. Zusammensetzung oder Kombination nach einem der vorhergehenden Ansprüche, wobei das Hydrogel eine wässrige Lösung ist, umfassend Gelatine, Alginat, Kollagen oder Mischungen davon.

7. Zusammensetzung oder Kombination nach einem der vorhergehenden Ansprüche, ferner umfassend ein keramisches Material, umfassend Calcium.

8. Zusammensetzung oder Kombination nach dem vorhergehenden Anspruch, wobei das keramische Material, umfassend Calcium, aus Calciumphosphat, Hydroxylapatit, calciummangelndem Hydroxylapatit, Brushit, Fluorapatit, Calcium-Natrium- und - Kaliumphosphat, Calcium- und Natriumphosphat, Calcium- und Kaliumphosphat, Calciumpyrophosphat, Calciumcarbonat, Calciumsulfat, Calciumsulfathemihydrat, Calciumoxid, Calciumhydroxid und Mischungen davon ausgewählt ist, wobei das keramische Material bevorzugt Hydroxylapatit, Brushit, Tricalciumphosphat oder Mischungen davon ist.

9. Zusammensetzung oder Kombination nach einem der vorhergehenden Ansprüche, wobei der STAT3-Inhibitor STAT3-Expression, STAT3-Phosphorylierung, STAT3-Dimerisierung, STAT3-Translokation zum Zellkern, STAT3-DNA-Bindung oder STAT3-vermittelte Transkription verhindert.

10. Zusammensetzung oder Kombination nach einem der vorhergehenden Ansprüche, wobei der STAT3-Inhibitor STAT3-Phosphorylierung verhindert, bevorzugter der STAT3-Inhibitor STAT3-Phosphorylierung an Tyrosin 705 verhindert.

11. Zusammensetzung oder Kombination nach einem der vorhergehenden Ansprüche, wobei der STAT3-Inhibitor S3I-201 oder BBI608 ist.

12. Zusammensetzung oder Kombination nach einem der vorhergehenden Ansprüche, ferner umfassend mindestens einen aktiven Wirkstoff, der aus einem chemotherapeutischen Wirkstoff und einem immuntherapeutischen Wirkstoff ausgewählt ist.

13. Zusammensetzung oder Kombination nach einem der vorhergehenden Ansprüche zur Verwendung bei der Behandlung von Krebs.

14. Zusammensetzung oder Kombination zur Verwendung nach dem vorhergehenden Anspruch, wobei der Krebs aus Knochenkrebs, Prostatakrebs, Brustkrebs, Gehirnkrebs oder Dickdarmkrebs ausgewählt ist.

15. Zusammensetzung oder Kombination zur Verwendung nach dem vorhergehenden Anspruch, wobei der Knochenkrebs Osteosarkom ist.

16. Zusammensetzung oder Kombination zur Verwendung nach einem der Ansprüche 13 bis 15, wobei die Verabreichung der Flüssigkeit oder eines Hydrogels, umfassend reaktive Sauerstoff- und Stickstoffspezies, gleichzeitig oder sequenziell in Bezug auf die Verabreichung des STAT3-Inhibitors ist.

## Revendications

1. Composition ou combinaison comprenant :
a. un liquide ou un hydrogel comprenant des espèces réactives de l'oxygène et de l'azote (RONS) ; et
b. un inhibiteur de STAT3.

2. Composition ou combinaison selon la revendication précédente, dans laquelle les RONS comprennent entre 10 et 3000 µM H₂O₂, préférablement entre 10 et 600 µM H₂O₂, plus préférablement entre 10 et 300 µM H₂O₂, encore plus préférablement entre 20 et 250 µM H₂O₂.

3. Composition ou combinaison selon l'une quelconque des revendications précédentes, dans laquelle les RONS comprennent entre 10 et 800 µM NO₂, préférablement entre 10 et 400 µM NO₂, plus préférablement entre 10 et 250 µM NO₂, encore plus préférablement entre 20 et 250 µM NO₂.

4. Composition ou combinaison selon l'une quelconque des revendications précédentes, dans laquelle le liquide est un milieu aqueux.

5. Composition ou combinaison selon l'une quelconque des revendications précédentes, dans laquelle l'hydrogel est une solution aqueuse comprenant au moins un élément parmi la gélatine, un dérivé de gélatine tel que la gélatine méthacrylée, la fibrine, la fibronectine, le collagène, un dérivé de collagène, l'alginate, l'agarose, la cellulose, la cellulose modifiée telle que l'hydroxypropylcellulose, la carboxyméthylcellulose ou l'hydroxyéthylcellulose, la gomme xanthane, le polyéthylèneglycol, l'acide hyaluronique, le chitosane, le polylactide-co-glycolide, les polyhydroxyalcanoates .

6. Composition ou combinaison selon l'une quelconque des revendications précédentes, dans laquelle l'hydrogel est une solution aqueuse comprenant la gélatine, l'alginate, le collagène ou des mélanges de ceux-ci.

7. Composition ou combinaison selon l'une quelconque des revendications précédentes, comprenant en outre un matériau céramique comprenant le calcium.

8. Composition ou combinaison selon la revendication précédente, dans laquelle le matériau céramique comprenant le calcium est choisi parmi le phosphate de calcium, l'hydroxyapatite, l'hydroxyapatite déficiente en calcium, la brushite, la fluorapatite, le phosphate de calcium-sodium et de potassium, le phosphate de calcium et de sodium, le phosphate de calcium et de potassium, le pyrophosphate de calcium, le carbonate de calcium, le sulfate de calcium, le sulfate de calcium hémihydraté, l'oxyde de calcium, l'hydroxyde de calcium et leurs mélanges, de préférence le matériau céramique est l'hydroxyapatite, la brushite, le phosphate tricalcique ou leurs mélanges.

9. Composition ou combinaison selon l'une quelconque des revendications précédentes, dans laquelle l'inhibiteur de STAT3 empêche l'expression de STAT3, la phosphorylation de STAT3, la dimérisation de STAT3, la translocation de STAT3 vers le noyau, la liaison à l'ADN de STAT3 ou la transcription médiée par STAT3.

10. Composition ou combinaison selon l'une quelconque des revendications précédentes, dans laquelle l'inhibiteur de STAT3 empêche la phosphorylation de STAT3, plus préférablement l'inhibiteur de STAT3 empêche la phosphorylation de STAT3 au niveau de la tyrosine 705.

11. Composition ou combinaison selon l'une quelconque des revendications précédentes, dans laquelle l'inhibiteur de STAT3 est S31-201 ou BBI608.

12. Composition ou combinaison selon l'une quelconque des revendications précédentes, comprenant en outre au moins un agent actif choisi parmi un agent chimiothérapeutique et un agent immunothérapeutique.

13. Composition ou combinaison selon l'une quelconque des revendications précédentes à utiliser dans le traitement du cancer.

14. Composition ou combinaison à utiliser selon la revendication précédente, dans laquelle le cancer est choisi parmi le cancer des os, le cancer de la prostate, le cancer du sein, le cancer du cerveau ou le cancer du côlon.

15. Composition ou combinaison à utiliser selon la revendication précédente, dans laquelle le cancer des os est l'ostéosarcome.

16. Composition ou combinaison à utiliser selon l'une quelconque des revendications 13 à 15, dans laquelle l'administration du liquide ou d'un hydrogel comprenant des espèces réactives de l'oxygène et de l'azote est simultanée ou séquentielle par rapport à l'administration de l'inhibiteur de STAT3.
